# EUROPÄISCHE PATENTSCHRIFT

(11) **EP 1 600 118 B1**
(45) Veröffentlichungstag und Bekanntmachung des Hinweises auf die Patenterteilung: **12.12.2007**
(21) Anmeldenummer: 05011117.8
(22) Anmeldetag: 23.05.2005
(51) Int. Cl.: A61F 2/00

(54) **Implantat zur Suspension der Harnblase bei Harninkontinenz der Frau**
Implant for the suspension of the urinary bladder for correction of female urinary incontinence
Implant pour suspension de la vessie dans les cas d'incontinence urinaire chez la femme

(30) Priorität: 24.05.2004 DE 102004025404
(43) Veröffentlichungstag der Anmeldung: 30.11.2005
(73) Patentinhaber: Serag-Wiessner KG, 95119 Naila (DE)
(72) Erfinder: Gramalla, Oliver, 95152 Selbitz (DE)
(74) Vertreter: Klingseisen, Franz

(56) Entgegenhaltungen:
- EP-A- 0 774 240
- EP-A- 1 382 728
- WO-A-03/096929
- DE-A1- 19 613 317
- DE-C- 703 123

## Beschreibung

Die Erfindung betrifft ein Implantat nach dem Oberbegriff des Anspruchs 1.

Ein Implantat dieser Art ist aus WO/03/096929 bekannt, wobei die seitlich abstehenden bandförmigen Fortsätze mit einer Umhüllung versehen sind, damit sich diese bandförmigen Fortsätze bei Zugbeanspruchung nicht einkringeln können und die Fortsätze durch die Umhüllung formstabil bleiben.

Aus DE 195 44 162 ist ein Implantat bekannt, das eine dreieckähnliche bis länglich ovale Basis aufweist, wobei auf gegenüberliegenden Seiten der Längsachse der Basis jeweils zwei Fortsätze entgegengesetzt zueinander so verlaufen, dass sie etwa parallel zur Längsachse der Basis liegen. Dieses Implantat besteht aus einem Polypropylennetz mit einer porösen Beschichtung aus einem resorbierbaren Kompositmaterial auf beiden Seiten. Beim Implantieren ist eine Eröffnung der Gewebeschichten im Körper erforderlich. Dieses in offener Chirurgie einzusetzende Implantat erfordert einen relativ aufwendigen chirurgischen Eingriff, der für die Patientin belastend ist.

Weiterhin ist aus DE 100 56 169 ein Implantat zum Halten der weiblichen Harnblase bekannt, das eine etwa länglich ovale Basis mit zwei beabstandeten Fortsätzen auf einer Schmalseite der Basis aufweist, wobei ein bandförmiger Träger durch die Enden dieser Fortsätze quer zur Längsachse der Basis derart verläuft, dass zwischen den beiden Fortsätzen ein Verbindungsabschnitt des Trägers liegt, der mit der Basis einen Freiraum zwischen den Fortsätzen begrenzt. In einem Abstand zu diesem bandförmigen Träger verläuft ein weiterer bandförmiger Träger quer zur Längsachse, der durch den gegenüberliegenden Endbereich der Basis geführt ist. Die Basis mit den beiden U-förmig angeordneten Fortsätzen besteht aus einem Gewirke aus Polypropylen, wobei die bandförmigen Träger durch Schlitze in der Basis geführt und aus Polypropylen gefertigt sind. Dadurch, dass Basis und Träger aus Einzelteilen bestehen, schrumpfen diese im Körper unterschiedlich, so dass es zu Komplikationen kommen kann.

Der Erfindung liegt die Aufgabe zugrunde, ein Implantat der eingangs angegebenen Art so auszugestalten, dass es unter minimalinvasiven Bedingungen implantiert werden kann.

Dies wird erfindungsgemäß durch die Merkmale im Anspruch 1 erreicht. Dadurch, dass das Implantat durchgehend die gleiche Bindungsstruktur aufweist und bandförmige Fortsätze seitlich von einer länglichen Basis abstehen, ergibt sich eine Ausgestaltung, die minimalinvasiv implantiert werden kann, wobei durch die Formgebung des Implantats und dessen Formstabilität eine optimale Abstützung der Harnblase erreicht wird.

Beispielsweise Ausführungsformen der Erfindung werden nachfolgend anhand der Zeichnung näher erläutert. Es zeigen
- Fig. 1: schematisch die Umrissform des flächigen Implantats in Form eines grobmaschigen Netzes,
- Fig. 2: die Abbildung einer durch Klöppeltechnik hergestellten praktischen Ausführungsform,
- Fig. 3: schematisch die Art der Fadenführung bei der Ausführungsform nach Fig. 2,
- Fig. 4: im Detail die Verbindungsstellen der sich kreuzenden Stränge,
- Fig. 5: das Bindungsbild der Klöppelstruktur nach Fig. 2,
- Fig. 6: eine andere Formgebung des Implantats, und
- Fig. 7: eine weitere Ausführungsform, und
- Fig. 8: eine Darsxtellung der Bindungsart durch Klöppeln.

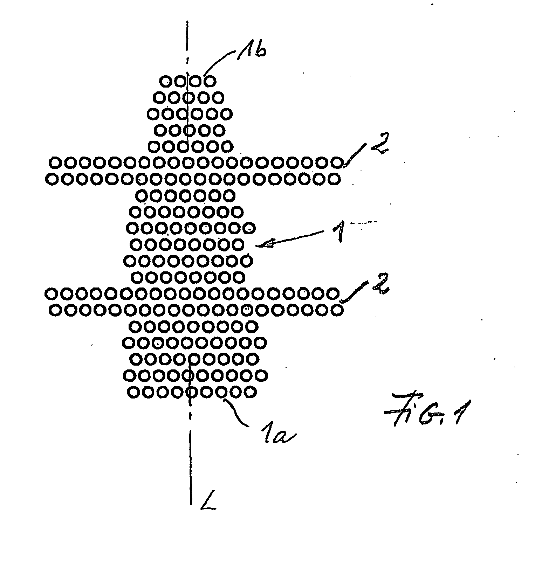

Fig. 1 zeigt schematisch die Umrissform des flächigen Implantats mit einer langgestreckt rechteckigen bzw. leicht trapezförmigen Basis 1, die in der Darstellung nach Fig. 1 unten eine längere Schmalseite 1a aufweist, während die obere Schmalseite 1b kürzer ausgebildet ist. Von den beiden Längsseiten gehen bandförmige Fortsätze 2 aus, die sich quer zur Längsachse L der Basis 1 in einem Abstand voneinander erstrecken, wobei sie auch einen Abstand von den Enden bzw. Schmalseiten der Basis haben. In der Praxis sind die Fortsätze 2 länger im Verhältnis zur Abmessung der Basis 1 ausgebildet als in Fig. 1 dargestellt.

In Fig. 1 ist die Grundstruktur des flächigen Implantats durch nebeneinander liegende Kreise schematisch wiedergegeben, wobei die Kreise eine grobmaschige Netzstruktur darstellen. Die bandförmigen Fortsätze 2 sind integral mit der Basis 1 ausgebildet, wobei sich die Netzstruktur der Basis 1 an den Fortsätzen 2 quer zur Längsachse L der Basis 1 fortsetzt.

Die grobmaschige Netzstruktur, aus der die Basis und die bandförmigen Fortsätze integral ausgebildet sind, kann in verschiedener Weise ausgebildet sein. Beispielsweise kann ein Gelege verwendet werden, wobei Monofilamente übereinandergelegt und miteinander verschmolzen werden. Es kann auch ein Gewebe vorgesehen sein, beispielsweise in Leinwandbindung. Weiterhin kann eine Maschenware vorgesehen werden, insbesondere eine Marquisette-Bindung, durch die ebenfalls eine ausreichende Porengröße erzielbar ist. Auch ist ein Vliesstoff möglich, wobei allerdings die Porengröße relativ klein ist. Bei all diesen Flächengebilden kommt es darauf an, dass bei Zug an einer Seite die Kraft entlang einzelner Längsfäden aufgenommen wird oder, wie beim Vliesstoff, multidirektional verteilt wird. Die in Fig. 1 wiedergegebene Umrissform des Implantats wird bei diesen Ausgestaltungen des Flächengebildes durch Zuschnitt aus der flächigen Textilbahn erreicht, wodurch allerdings abstehende Fäden an den Rändern entstehen.

Vorteilhafterweise wird das das Implantat bildende Flächengebilde durch Klöppeltechnik hergestellt, wodurch es möglich ist, an den Rändern abstehende Fäden zu vermeiden, weil ein Zuschnitt nicht erforderlich ist. Durch Klöpplen kann jede erwünschte, an die anatomischen Gegebenheiten angepasste Form des Implantats mit glatten Rändern erhalten werden.

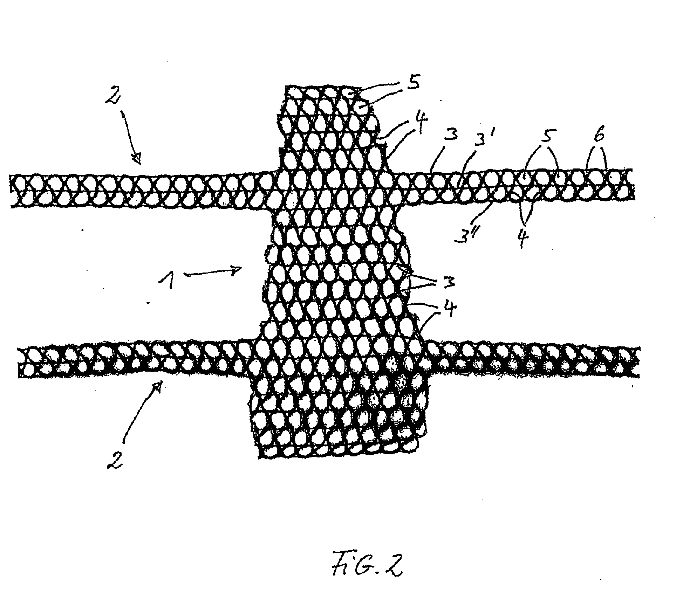

Fig. 2 zeigt eine Abbildung einer praktischen Ausführungsform eines Implantats, das durch Klöppeln hergestellt ist. Zwischen quer zur Längsachse verlaufenden, im Wesentlichen geraden Strängen 3 sind durch zwei Gruppen von schräg dazu verlaufenden Strängen 4 etwa kreisförmige Freiräume 5 ausgebildet, die in etwa der Darstellung in Fig. 1 entsprechen. Die bandförmige Fortsätze 2 werden bei diesem Ausführungsbeispiel durch drei etwa parallel zueinander verlaufenden Strängen 3, 3' und 3" gebildet, die durch die Basis 1 verlaufen, wobei zwischen den geraden Strängen 3 im Bereich der Fortsätze 2 etwa sinusförmig verlaufende Stränge 4 eingebunden sind, die im Bereich der Basis 1 schräg zur Längsachse der Basis verlaufen.

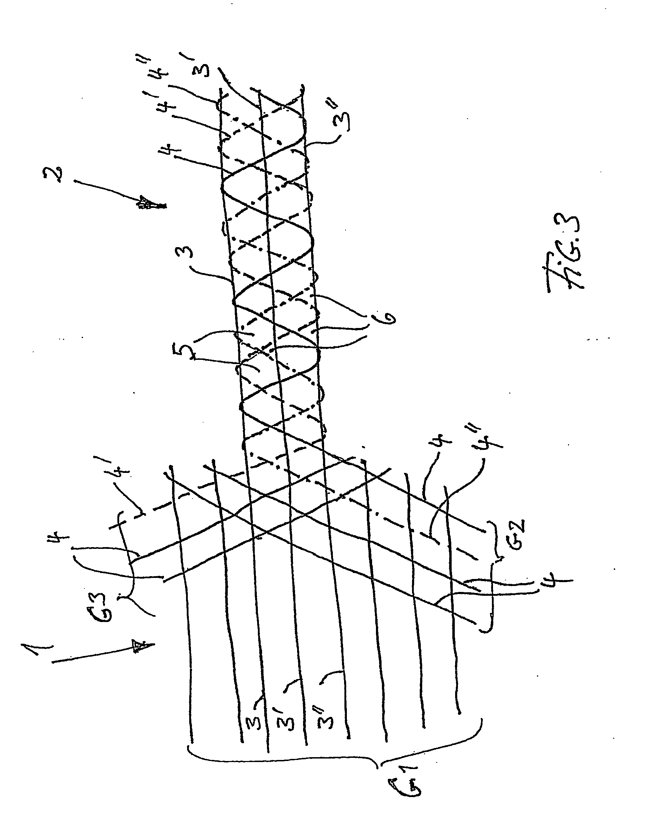

Fig. 3 zeigt schematisch den Verlauf der Stränge 3 und 4 im Übergangsbereich zwischen einem Fortsatz 2 und der Basis 1, wobei zur Verdeutlichung der Darstellung die im Bereich der Fortsätze 2 etwa sinusförmig verlaufenden und sich überschneidenden Stränge durch eine ausgezogene Linie 4, durch eine gestrichelte Linie 4' und durch eine strichpunktierte Linie 4" wiedergegeben sind, während die im Wesentlichen gerade verlaufenden Stränge 3 mit einer durchgehenden Linie wiedergegeben sind. Die etwa sinusförmig zwischen den äußeren Strängen 3 und 3" verlaufenden Stränge 4, 4' und 4" überschneiden sich derart, dass jeweils zwischen zwei geraden Strängen 3 und 3' bzw. 3' und 3" eine Kreuzungsstelle der sinusförmig verlaufenden Stränge 4, 4' und 4" auftritt. Beim Übergang von einem Fortsatz 2 in die Basis 1 verlaufen diese Stränge 4, 4' und 4" schräg zu den etwa parallel angeordneten geraden Strängen 3 bzw. schräg zur Längsachse der Basis 1. Zwischen den sich kreuzenden, sinusförmig verlaufenden Strängen 4, 4' und 4" und den geraden Strängen 3 und 3' bzw. 3" werden die in Fig. 1 und 2 etwa kreisförmig wiedergegebenen Freiräume 5 ausgebildet, wobei dazwischen etwa dreieckige Freiräume 6 verbleiben. Diese dreieckigen Freiräume 6 sind längs des mittleren Stranges 3' abwechselnd auf der einen und der anderen Seite ausgebildet, während sie längs der äußeren Stränge 3 und 3" jeweils zwischen zwei etwa kreisförmigen Freiräumen 5 ausgebildet sind. In gleicher Weise sind im Bereich der Basis 1 diese etwa dreieckigen Freiräume 6 zwischen den etwa kreisförmigen Freiräumen 5 ausgebildet, wie dies auch Fig. 2 zeigt.

Die in den Fig. 2 und 3 wiedergegebene Struktur eines Flächengebildes ist durch drei Gruppen von Strängen ausgebildet. Eine erste Gruppe G 1 wird durch die quer zur Längsachse der Basis 1 verlaufenden Stränge 3 gebildet, eine zweite Gruppe G2 durch Stränge 4, die im Bereich der Basis 1 in Fig. 3 von links unten schräg nach rechts oben verlaufen, und durch eine dritte Gruppe G3 von Strängen 4, die in Fig. 3 von links oben schräg nach rechts unten verlaufen. Diese beiden Gruppen G2 und G3 schneiden sich derart, dass dazwischen rautenförmige Freiräume gebildet werden, wobei die Eckpunkte der Rauten zwischen den Strängen 3 der ersten Gruppe G1 liegen. Im Bereich eines bandförmigen Fortsatzes 2 werden bei dem Ausführungsbeispiel nach Fig. 3 die sinusförmig verlaufenden Stränge durch zwei Stränge 4 und 4" der Gruppe G2 und durch einen Strang 4' der Gruppe G3 gebildet, wobei wiederum wie im Bereich der Basis 1 die Kreuzungsstellen der Stränge 4 zwischen den Strängen 3 liegen.

Die einzelnen Stränge 3 und 4 bestehen jeweils aus zwei in sich verdrillten Monofilamenten bzw. Fäden. An jeder Kreuzungsstelle sind die Stränge 3 und 4 derart miteinander verbunden, dass ein Faden des einen Strangs zwischen den eine Schlaufe bildenden beiden Fäden des anderen Strangs verläuft und umgekehrt. Hierdurch ergibt sich eine Fixierung der Kreuzungsstellen im Gesamtaufbau des Flächengebildes, so dass dieses die in Fig. 2 wiedergegebene Struktur auch unter Beanspruchung beibehält. Bei Zug an den bandförmigen Fortsätzen 2 tritt kein Einrollen auf, weil die geraden Stränge 3 die Zugkraft aufnehmen. Das Band bleibt vollflächig stabil.

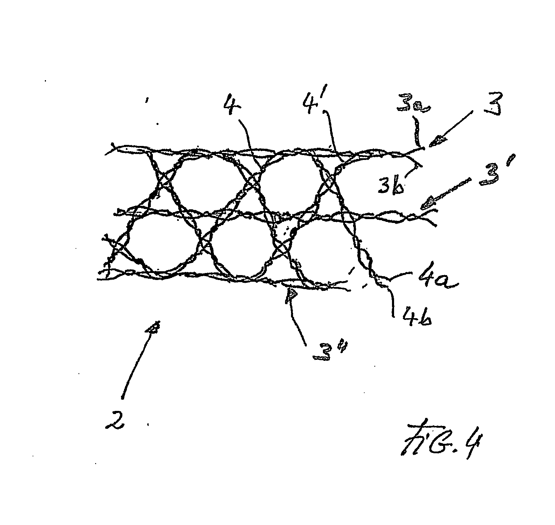

Fig. 4 zeigt im Einzelnen die Art der Verbindung zwischen den Strängen 3 und 4. Die horizontal gerade verlaufenden Stränge 3 bestehen aus zwei Fäden 3a und 3b, die miteinander verdrillt sind. In der gleichen Weise sind die Stränge 4 ausgebildet. Bei dem dargestellten Ausführungsbeispiel bildet die Verdrillung abwechselnd eine große Schlaufe und eine kleine Schlaufe zwischen den einzelnen Verdrillungsstellen. Die Stränge 4 und 4' kreuzen sich jeweils im Bereich einer großen Schlaufe derart, dass z. B. der eine Faden 4a des Stranges 4 durch eine größere Schlaufe zwischen den Fäden 4'a und 4'b des Stranges 4' verläuft, während ein Faden 4'a durch die von den Fäden 4a und 4b gebildete Schlaufe verläuft. In der gleichen Weise sind die Stränge 3 mit den Strängen 4 verbunden, wie dies Fig. 4 an den Kreuzungsstellen zwischen dem Strang 3' und den Strängen 4 und 4' zeigt.

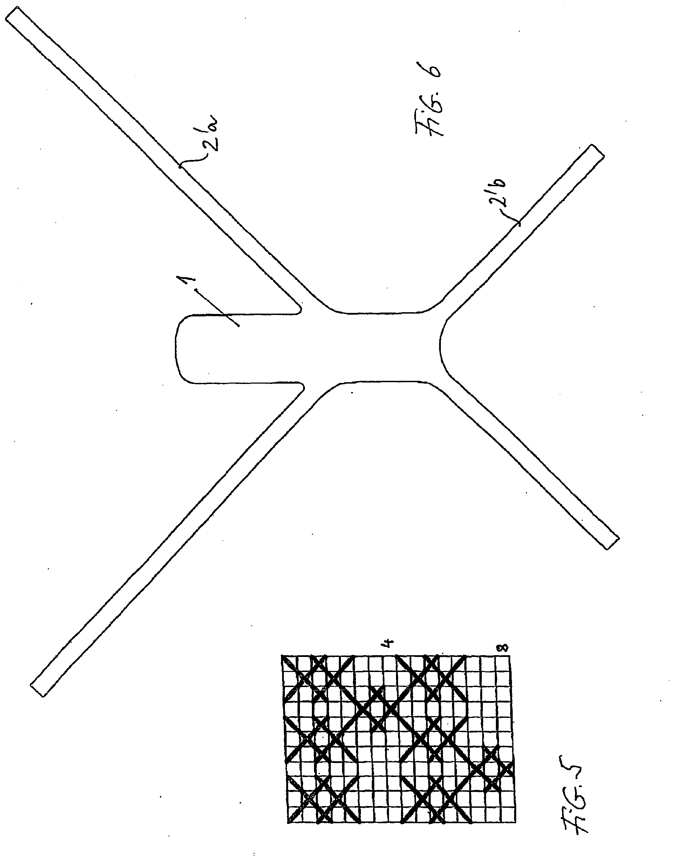

Fig. 5 zeigt ein Grundbindungsbild der textilen Klöppelstruktur (11 x 8 = 88 Klöppel) entsprechend dem Ausführungsbeispiel nach Fig. 2.

Durch die in Richtung der bandförmigen Fortsätze 2 verlaufenden, geraden Stränge 3 ergibt sich in dieser Richtung eine hohe Zugfestigkeit, während quer zu den Strängen 3 das Flächengebilde stauchbar und auch etwas dehnbar ist. Damit unterscheiden sich die Eigenschaften des Flächengebildes in Richtung längs und quer relativ zur Längsachse der Basis. Die Bindungsart ist im gesamten Bereich des Flächengebildes die gleiche.

Die Einzelfäden 3a, 3b und 4a, 4b der Stränge 3 und 4 können aus PP (Polypropylen), das auch durch weitere Materialzugaben variiert werden kann, aus PVDF (Polyvinylidendifluorid), PET (Polyethylenterephthalat), PA (Polyamid), PCL/PGA (Copolymer aus Polycaprolactan und Polyglycolacid) oder Mischungen daraus bestehen.

Anstelle der monofilen Fäden 3a, 3b bzw. 4a, 4b können auch multifile Fäden für die Stränge 3 und 4 verwendet werden, wobei monifile Fäden eine geringere Gefahr von Bewuchs durch Bakterien aufweisen. Diese Gefahr kann durch eine Fadenbeschichtung mit einer antimikrobiell wirkenden Substanz umgangen werden. Es ist auch eine Kombination aus monofilen und multifilen Fäden möglich.

Insgesamt ergibt sich ein leichtgewichtiges und grobmaschiges Netz, wodurch das Einwachsen des Implantats im Körper begünstigt wird. Hierzu trägt auch die Formstabilität der Grundstruktur des Flächengebildes bei.

Das beschriebene Ausführungsbeispiel mit monofilen Fäden zur Ausbildung der Stränge 3 und 4 hat vorzugsweise ein Flächengewicht von 20 bis 40, insbesondere von 30g/m² bei einer Poren- bzw. Maschengröße von etwa 4 x 4 mm. Die Biegesteifigkeit beträgt in Längsrichtung der bandförmigen Fortsätze etwa 40 mg und quer dazu bzw. in Richtung der Längsachse der Basis etwa 20 mg. Der Abstand der geraden Stränge 3 voneinander sowie der Abstand der Stränge 4 voneinander beträgt etwa 4 bis 5 mm.

Die monofilen Fäden haben vorzugsweise einen Durchmesser von 0,1 bis 0,2, insbesondere von 0,15 mm. Hierdurch ergibt sich eine Dicke des Implantats von etwa 0,2 - 0,3 mm. Die Festigkeit des so ausgebildeten Netzes liegt vorzugsweise bei 45 bis 60 N und die Dehnung beträgt 20 bis 60 %.

Die die Stränge bildenden Fäden können aus einem monofilen Polypropylenfaden der Stärke EP 1,5 (Europäische Pharmakopöe) bzw. USP 5/0 mit einer Biegesteifigkeit von 2 mg bestehen.

Je nach Verwendungszweck kann die Poren- bzw. Maschengröße auch im Bereich von 2 x 2 bis 4 x 4 mm und darüber ausgebildet werden.

Trotz der grobmaschigen Grundstruktur wird durch die beschriebene Verbindung der Stränge an den Kreuzungsstellen durch Klöppeltechnik ein relativ steifes bzw. rigides und formstabiles Flächengebilde erreicht.

Die beschriebene Netzstruktur des Flächengebildes stellt eine neuartige Eigenschaftsmischung für ein textiles Implantat dar, wobei innerhalb einer Materialart ein leichtgewichtiges, dennoch monofiles und gleichzeitig grobmaschiges Netz ausgebildet wird, das eine für ein Implantat wünschenswerte Rigidität und Formstabilität hat. Durch die Verwendung von sehr dünnen Einzelfäden besitzt das Implantat eine patientenverträgliche Weichheit. Damit werden durch die beschriebene Ausführungsform die technologischen Idealforderungen an ein Implantat erfüllt.

Das beschriebene Flächengebilde eignet sich für verschiedene Implantate. So kann es auch in der Hernienchirurgie eingesetzt werden, wobei keine bandförmigen Fortsätze 2 benötigt werden.

Zur besseren Wiederfindung im Gewebe ist es zweckmäßig, die Hauptfläche des Implantats farbig zu gestalten. Ungefärbte oder andersfarbige Fadensysteme dienen lediglich der vereinfachten symmetrischen Fixierung des Implantats im Körper. So können beispielsweise Markierungsfäden entlang der bandförmigen Fortsätze in weiß oder einer anderen Farbe unterschiedlich zum übrigen Aufbau des Implantats vorgesehen werden.

In das beschriebene Flächengebilde können weitere Materialien mit eingearbeitet werden, die zusätzliche technologische oder medizinfunktionale Effekte haben. So können einzelne Stränge oder Fäden auch aus resorbierbarem Material bestehen. Ebenso ist es möglich, das Flächengebilde mit einer Beschichtung auszustatten.

Die monofilen Fäden, aus denen die Stränge 3 und 4 ausgebildet sind, können auch aus Bikomponentenfasern bestehen, wobei ein Monofilament aus zwei Einzelteilen besteht. Hierbei kann eine Hälfte aus Polypropylen und die andere Hälfte aus einem resorbierbaren Material, z. B. Monocryl bestehen. Hierdurch können für die Implantation optimale Eigenschaften des Netzes vorgegeben werden, während nach der Implantation ein leichtgewichtiges Netz verbleibt, wenn der resorbierbare Anteil abgebaut ist. In den resorbierbaren Anteil können auch Arzneimittel eingebracht werden, die dann im Körper kontrolliert freigesetzt werden.

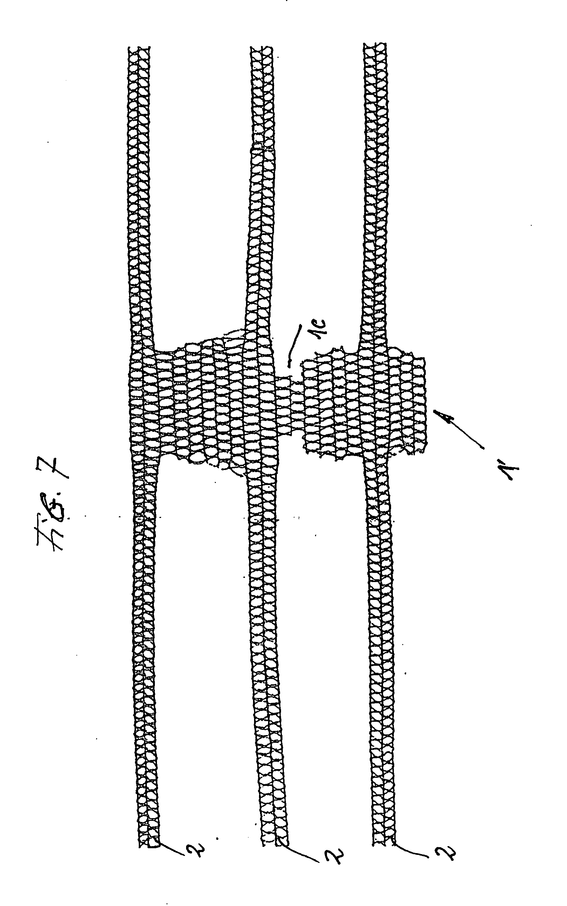

Fig. 6 zeigt eine abgewandelte Ausführungsform des Implantats mit schräg von der langgestreckt rechteckigen Basis 1 abstehenden, bandförmigen Fortsätzen 2', wobei die oberen, schräg zur Längsachse der Basis abstehenden Fortsätze 2'a etwa vom Mittelbereich der Basis 1 ausgehen, während die unteren etwa im gleichen Winkel gegenläufig abstehenden Fortsätze 2'b vom unteren Ende der Basis 1 ausgehen, so dass sich insgesamt etwa ein Umrissbild eines Frosches in stilisierter Form ergibt.

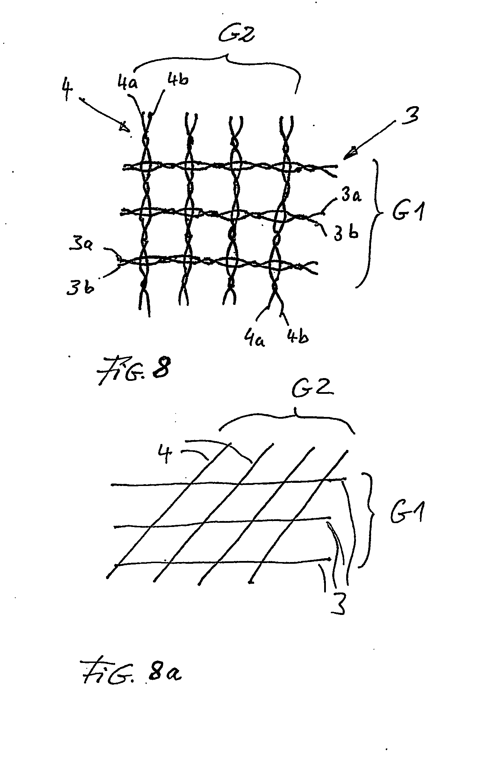

Fig. 7 zeigt eine weitere Ausführungsform eines Implantats mit drei Paaren von einander gegenüberliegenden Fortsätzen 2, die etwa senkrecht zur Längsachse der langgestreckten Basis 1' seitlich abstehen. Wie dargestellt kann die Basis 1' im Mittelbereich entsprechend den anatomischen Gegebenheiten eine Einschnürung 1 c aufweisen angrenzend an die in der Mitte liegenden Fortsätze 2. Bei dieser Ausführungsform sind die in Fig. 7 oberen Fortsätze 2 am Rand der Basis 1' angesetzt.

Entsprechend der erfindungsgemäßen Ausgestaltung können zwei, vier, sechs oder mehrere bandförmige Fortsätze 2 von der anatomisch geformten Basis 1 bzw. 1' quer zur Längsachse in einem Abstand voneinander abstehen, die zugstabil sind und die gleiche Bindungsart aufweisen wie die Basis, mit der sie aus einem Stück bestehen.

Fig. 8 zeigt zur Verdeutlichung der Bindungsart beim Klöppeln einen Ausschnitt aus einem Flächengebilde, das aus zwei Gruppen G1 und G2 von sich kreuzenden Strängen 3 und 4 ausgebildet ist, wobei die einzelnen Stränge aus wenigstens zwei Fäden 3a und 3b bzw. 4a und 4b bestehen, die in sich verdrillt sind, so dass sich zwischen den Verdrillungsstellen Schlaufen ergeben. Bei dem dargestellten Ausführungsbeispiel liegen zwischen jeweils zwei größeren Schlaufen kleinere Schlaufen, wobei die größeren Schlaufen an den Kreuzungsstellen der Stränge 3 und 4 liegen. Die Stränge 3 und 4 sind derart miteinander verbunden, dass z. B. der eine Faden 4a eines Stranges 4 durch eine größere Schlaufe eines Stranges 3 zwischen dessen Fäden 3a und 3b verläuft, während ein Faden 3a durch die von den Fäden 4a und 4b gebildete Schlaufe eines Stranges 4 verläuft. Auf diese Weise ergibt sich ein formstabiles Netz, weil die Kreuzungs- und Verbindungsstellen in ihre Lage zwischen den Verdrillungsstellen der einzelnen Stränge fixiert sind.

Diese Bindungsart wird vorzugsweise durch Klöppeln hergestellt, wobei verschiedene Abwandlungen in der Struktur des Flächengebildes möglich sind. Bei dem Ausführungsbeispiel nach Fig. 1 mit etwa quadratischen Maschen kann der Abstand zwischen den etwa parallel zueinander verlaufenden Strängen 3 und 4 variiert werden, um die Maschengröße des Netzes an die jeweiligen Erfordernisse anzupassen. Es ist auch möglich, die Stränge 4 schräg zu den Strängen 3 anzuordnen, wie dies Fig. 8a schematisch zeigt, so dass sich rautenförmige Maschen zwischen den beiden Gruppen von Strängen ergeben.

Wie die Figuren zeigen, ist durch Klöppeltechnik eine vielfältige Struktur des Flächengebildes möglich, so dass nicht nur die Struktur des Flächengebildes selbst entsprechend den Anforderungen grob- oder kleinmaschig mit unterschiedlichen Formen der Maschen ausgelegt werden kann, sondern auch Fortsätze an einem Flächengebilde integral mit dem Flächengebilde entsprechend den jeweiligen Anforderungen ausgebildet werden können.

## Patentansprüche

1. Implantat zur Suspension der Harnblase bei Senkungsbeschwerden und/oder bei Harninkontinenz der Frau, umfassend
eine etwa rechteckige, ovale oder langgestreckt trapezförmige Basis (1), von deren Längsseiten in einem Abstand voneinander bandförmige Fortsätze (2) abstehen, wobei die bandförmigen Fortsätze (2) die gleiche Bindungsart aufweisen wie die Basis (1) und mit dieser aus einem Stück bestehen,
**dadurch gekennzeichnet,**
**daß** quer zur Längsachse der Basis (1) Stränge (3) verlaufen, die im Bereich der bandförmigen Fortsätze (2) sich längs diesen erstrecken, wobei diese Stränge (3) durch quer dazu verlaufende Stränge (4) miteinander verbunden sind.

2. Implantat nach Anspruch 1, wobei die quer zur Längsachse der Basis verlaufenden Stränge (3) eine erste Gruppe (G1) bilden, deren Stränge von den Strängen (4) einer zweiten und dritten Gruppe miteinander verbunden sind, die entgegengesetzt schräg zur Längsachse der Basis verlaufen, wobei einzelne Stränge (4, 4', 4") dieser beiden Gruppen (G2, G3) sinusförmig im Bereich der Fortsätze (2) geführt sind, um die Stränge (3) der ersten Gruppe (G1) miteinander zu verbinden, von denen zwei Stränge die bandförmigen Fortsätze (2) begrenzen.

3. Implantat nach einem der vorhergehenden Ansprüche, wobei die Stränge (3, 4) jeweils aus miteinander verdrillten Fäden (3a, 3b; 4a, 4b) bestehen und an den Kreuzungsstellen **dadurch** miteinander verbunden sind, dass ein Faden des einen Stranges zwischen den Fäden des anderen Stranges zwischen zwei Verdrillungsstellen verläuft.

4. Implantat nach einem der vorhergehenden Ansprüche, wobei die entgegengesetzt schräg zur Längsachse der Basis (1) verlaufenden Stränge (4) Rauten begrenzen, deren Ecken zwischen den Strängen (3) der ersten Gruppe (G1) liegen.

5. Implantat nach einem der vorhergehenden Ansprüche, wobei die Stränge (3, 4) aus monofilen und/oder multifilen Fäden (3a, 3b; 4a, 4b) ausgebildet sind.

6. Implantat nach Anspruch 5, wobei die monofilen oder multifilen Fäden der Stränge (3, 4) aus Polypropylen, PVDF, PET, PA, PCL/PGA oder Mischungen daraus bestehen.

7. Implantat nach einem der vorhergehenden Ansprüche, wobei die die Stränge bildenden Fäden aus einem monofilen Polypropylenfaden der Stärke EP 1,5 mit einer Biegesteifigkeit von 2 mg bestehen.

8. Implantat nach einem der vorhergehenden Ansprüche, wobei sowohl die Basis (1, 1') wie auch die bandförmigen Fortsätze (2) aus einer grobmaschigen Textilstruktur gefertigt sind, die Maschen bzw. offene Bereiche mit einem Durchmesser von etwa 2 bis 5 mm bildet.

9. Implantat nach einem der vorhergehenden Ansprüche, wobei die Bindungsart durch Klöppeln ausgebildet ist.

## Claims

1. Implant for suspending the urinary bladder in the case of sagging and/or female aconuresis, comprising
an approximately rectangular, oval or longitudinal trapezoid base (1), from whose long sides band-shaped extensions (2) project at a distance from one another, wherein the band-shaped extensions (2) have the same type of weaving as the base (1) and consist of one piece with the base (1),
**characterized in that**
threads (3) extend transverse to the longitudinal axis of the base (1), extending along the band-shaped extensions (2) in the area thereof, wherein these threads (3) are joined to one another by threads (4) extending transversely thereto.

2. Implant according to claim 1, wherein the threads (3) extending transverse to the longitudinal axis of the base form a first group (G1) whose threads of the threads (4) of a second and third group are joined to one another, which extend opposite one another inclined to the longitudinal axis of the basis, wherein individual threads (4, 4', 4") of these two groups (G2, G3) are guided in a sine shape in the area of the extensions (2) for joining the threads (3) of the first group (G1) to one another, of which two threads define the band-shaped extensions (2).

3. Implant according to one of the preceding claims, wherein the threads (3, 4) each consist of strands twisted together (3a, 3b; 4a, 4b) and are joined to one another at the points of intersection in that one strand of the one thread extends between the strands of the other thread between two twisting points.

4. Implant according to one of the preceding claims, wherein the threads (4) extending opposite one another inclined to the longitudinal axis of the base (1) define rhombuses whose corners lie between the threads (3) of the first group (G1).

5. Implant according to one of the preceding claims, wherein the threads (3, 4) are formed from monofil and/or multifil strands (3a, 3b; 4a, 4b).

6. Implant according to claim 5, wherein the monofil or multifil strands of the threads (3, 4) consist of polypropylene, PVDF, PET, PA, PCL/PGA or mixtures thereof.

7. Implant according to one of the preceding claims, wherein the strands forming the threads consist of a monofil polypropylene strand with a strength of EP 1.5 and a flexural stiffness of 2 mg.

8. Implant according to one of the preceding claims, wherein both the base (1, 1') and the band-shaped extensions (2) are made of a coarse mesh textile structure, which forms stitches or open portions having a diameter of approximately 2 to 5 mm.

9. Implant according to one of the preceding claims, wherein the type of weaving is formed by tatting.

## Revendications

1. Implant pour la suspension de la vessie urinaire en cas de troubles de la statique pelvienne et/ou en cas d'incontinence urinaire chez la femme, comprenant une embase (1) sensiblement rectangulaire, ovale ou de forme trapézoïde allongée, des côtés longitudinaux de laquelle partent des prolongements (2) en forme de bande et à distance l'un de l'autre, lesdits prolongements (2) en forme de bande ayant le même type d'armure que l'embase (1) et étant solidaires de celle-ci,
**caractérisé en ce que**
des écheveaux (3) s'étendent transversalement par rapport à l'axe longitudinal de l'embase (1) et s'étendent, dans la zone des prolongements (2) en forme de bande, le long de ceux-ci, lesdits écheveaux (3) étant reliés les uns aux autres au moyen d'écheveaux (4) s'étendant transversalement par rapport à ceux-ci.

2. Implant selon la revendication 1, dans lequel les écheveaux (3) s'étendant transversalement par rapport à l'axe longitudinal de l'embase forment un premier groupe (G1) dont les écheveaux sont reliés les uns aux autres au moyen d'écheveaux (4) d'un deuxième et troisième groupes qui s'étendent dans des directions opposées de manière oblique par rapport à l'axe longitudinal de l'embase, des écheveaux (4, 4', 4") individuels de ces deux groupes (G2, G3) étant menés de manière sinusoïdale dans la zone des prolongements (2) pour relier les écheveaux (3) du premier groupe (G1) les uns aux autres, dont deux écheveaux délimitent les prolongements (2) en forme de bande.

3. Implant selon l'une des revendications précédentes, dans lequel les écheveaux (3, 4) sont chacun constitués de fils torsadés (3a, 3b ; 4a, 4b) et sont reliés les uns aux autres, au niveau des points de croisement, en ce sens qu'un fil de l'un écheveau s'étend, entre deux points de torsadage, entre les fils de l'autre écheveau.

4. Implant selon l'une des revendications précédentes, dans lequel les écheveaux (4) s'étendant dans des directions opposées de manière oblique par rapport à l'axe longitudinal de l'embase (1) délimitent des losanges dont les angles sont situés entre les écheveaux (3) du premier groupe (G1).

5. Implant selon l'une des revendications précédentes, dans lequel les écheveaux (3, 4) sont formés de fils monofilaments et/ou multifilaments (3a, 3b ; 4a, 4b).

6. Implant selon la revendication 5, dans lequel les fils monofilaments ou multifilaments des écheveaux (3, 4) sont constitués de polypropylène, PVDF, PET, PA, PCL/PGA ou des mélanges de ceux-ci.

7. Implant selon l'une des revendications précédentes, dans lequel les fils formant les écheveaux sont constitués d'un fil de polypropylène monofilament d'une épaisseur EP 1,5 avec une rigidité flexionnelle de 2 mg.

8. Implant selon l'une des revendications précédentes, dans lequel aussi bien l'embase (1, 1') que les prolongements (2) en forme de bande sont fabriqués à partir d'une structure textile à grosses mailles qui forme des mailles ou des régions ouvertes d'un diamètre compris entre environ 2 et 5 mm.

9. Implant selon l'une des revendications précédentes, dans lequel le type d'armure est réalisé au fuseau.
